**Europäisches Patentamt**

**European Patent Office**

**Office européen des brevets**

(11) Veröffentlichungsnummer: **0 160 815**
**A2**

(12) **EUROPÄISCHE PATENTANMELDUNG**

(21) Anmeldenummer: **85103185.6**

(22) Anmeldetag: **19.03.85**

(51) Int. Cl.⁴: **G 01 N 33/18**

(30) Priorität: **11.05.84 DE 3417454**

(43) Veröffentlichungstag der Anmeldung:
**13.11.85 Patentblatt 85/46**

(84) Benannte Vertragsstaaten:
**AT BE CH DE FR GB IT LI LU NL SE**

(71) Anmelder: **PIERBURG GmbH & Co. KG**
**Leuschstrasse 1**
**D-4040 Neuss 1(DE)**

(72) Erfinder: **Eichhorn, Bernd**
**Roonstrasse 24**
**D-4100 Duisburg 17(DE)**

(74) Vertreter: **Bergen, Klaus, Dipl.-Ing. et al,**
**Patentanwälte Dr.-Ing. Reimar König Dipl.-Ing. Klaus**
**Bergen Wilhelm-Tell-Strasse 14 Postfach 260162**
**D-4000 Düsseldorf 1(DE)**

(54) Vorrichtung zum Messen des Ölgehaltes von Wasser.

(57) Bei einer Vorrichtung zum Messen des Ölgehaltes von Wasser, bie dem aus einem Lösungsmittel- und einem Probenwasser-Do-sierbehälter (4, 5) je eine Menge eines Lösungsmittels und eine Menge Probenwasser zu einem Extraktor (32) gefördert wird, das Öl aus dem Probenwasserstrom mit einem organischen Lösungsmittel extrahiert, das Wasser in einem Trennfilter (43) abgetrennt, das ölbeladene Lösungsmittel in einem, insbesondere als Infrarot-Meßgerät arbeitenden, Analysator (45) analysiert, das Lösungsmittel nachfolgend von dem Öl abgetrennt und im Kreislauf wieder verwendet sowie das abgetrennte Wasser über eine Reinigungsstufe abgeführt wird, wird ein ein zuverlässiges Messen ermöglichendes, genaues Dosieren der Flüssigkeiten dadurch erreicht, daß als Überlaufbehälter ausgebildeten Dosierbehältern (4, 5) einer Dosiereinheit (2) mit genau festgelegtem Aufnahme- bzw. Volumenverhältnis ständig umlaufende, Lösungsmittel oder Probenwasser fördernde Pumpen (23, 24) mit zwischengeschalteten, aufsteuerbaren Sperreinrichtungen, vorzugsweise Mehrwegeventilen, (17, 18) in den Zuführleitungen zugeordnet sind.

Fig 1

Pierburg GmbH & Co KG, Leuschstraße 1,
=========================================

4040 Neuss 1
============

## "Vorrichtung zum Messen des Ölgehaltes von Wasser"

Die Erfindung betrifft eine Vorrichtung zum Messen des Ölgehaltes von Wasser, bei dem aus Dosierbehältern eine Menge eines Lösungsmittels und eine Menge Probenwasser zu einem Extraktor gefördert wird, das Öl aus dem Probenwasserstrom mit einem organischen Lösungsmittel extrahiert, das Wasser in einem Trennfilter abgetrennt, das ölbeladene Lösungsmittel in einem, insbesondere als Infrarot-Meßgerät arbeitenden, Analysator analysiert, das Lösungsmittel nachfolgend von dem Öl abgetrennt und im Kreislauf wieder verwendet sowie das abgetrennte Wasser über eine Reinigungsstufe abgeführt wird.

Derartige Vorrichtungen dienen der Überwachung öffentlicher Gewässer, die der Gefahr einer unzuträglichen Belastung mit Kohlenwasserstoffen ausgesetzt sind, wie z.B. die Entwässerungskanäle von Flugplätzen, oder sie dienen der Überwachung von Milchprodukten, der Überwachung der Kreislaufflüssigkeiten von Öl/Wasserwärmeaustauschern, der Kontrolle bei der Schneidölaufbereitung oder der Trinkwasserüberwachung (DE-Z.: "Bosch Techn. Berichte 2, Heft 5, November 1968"). Das regelmäßige Überwachen setzt zuverlässige und rasch durchzuführende Analysenmethoden voraus, damit sich Umfang und Herkunft von Verschmutzungen erfassen und Schäden verhüten lassen. Mit solchen Analysen müssen sich bis 1000 ppm Kohlenwasserstoffe im Wasser messen lassen.

Durch die DE-OS 1 810 604 ist ein Infrarot-Absorptionsverfahren zum Bestimmen der in einem zur Ölextraktion verwendeten Lösungsmittel enthaltenen Ölmenge durch Beobachtung der Lichtabsorption einer eingestrahlten Infrarotlichtmenge bekannt. Die Infrarot-Absorptionsmethode hat sich gegenüber anderen bekannten Verfahren wegen ihrer sehr hohen Empfindlichkeit überlegen erwiesen, wobei es sich insbesondere vorteilhaft auswirkt, daß die Empfindlichkeitsänderung aufgrund einer Änderung der Ölart relativ klein ist. Da jedoch das Vorhandensein der sonstigen Flüssigkeit, insbesondere von Wasser einen Meßfehler ergibt, ist es erforderlich, zunächst eine Extraktion mit einem organischen Lösungsmittel durchzuführen und die erhaltene Lösung zu trennen.

Um die geforderten zuverlässigen Meßwerte innerhalb des engen zulässigen Toleranzbereiches von 10 bis 50 mg/l erreichen zu können, müssen die dem Extraktor zugeführten Mengen des Lösungsmittels und des Probenwassers in einem genauen Verhältnis zueinander stehen, da damit der Meßbereich festgelegt wird. Bei dem bekannten Gerät sind dazu Turbulenzerzeuger und/oder Differenzdruck-Stabilisatoren zum Ausgleich schwankender Drücke in der Austrittsleitung des Probenwassers und der Lösungsmittelleitung vorgesehen.

Der Erfindung liegt daher die Aufgabe zugrunde, zum automatischen Betrieb eines Analysengerätes eine Dosiereinrichtung zu schaffen, mit der sich dem Extraktor insbesondere ohne zusätzliche Hilfseinrichtungen in einer quasi kontinuierlichen Betriebsweise genau abgestimmte Mengenanteile von Lösungsmittel und Probenwasser periodisch zuführen lassen.

Diese Aufgabe wird erfindungsgemäß dadurch gelöst, daß als Überlaufbehälter ausgebildeten Dosierbehältern einer Dosiereinheit mit genau festgelegtem Aufnahme- bzw. Volumenverhältnis ständig umlaufende, Lösungsmittel oder Probenwasser fördernde Pumpen mit zwischengeschalteten Sperreinrichtungen, vorzugsweise Mehrwegeventilen, zugeordnet sind. Indem die Behälter als Überlaufbehälter ausgeführt sind, - wenn sie voll sind, läuft das steigend in den jeweiligen Behäl-

- 3 -

0160815

ter eingefüllte Probenwasser bzw. Lösemittel über den oberen Rand des Behälters ab und kann danach die Anlage verlassen - ist gewährleistet, daß immer eine frische Probe vorhanden ist. Die Überlaufbehälter lassen sich in verschiedenen Bauformen ausführen, wobei sich jedoch auf jeden Fall zylindrische Behälter empfehlen.

Das Mischungsverhältnis läßt sich dadurch optimieren, daß an der Überlaufseite jedes Dosierbehälters ein Verdrängungskegel in die Flüssigkeits-Aufnahmekammer eintaucht.

Zum Fördern der Flüssigkeiten eignen sich vorteilhaft Schwingankerpumpen, wobei Näherungsschalter an den Dosierbehältern vorgesehen sein können, die beim Erreichen der erforderlichen Füllstandshöhe die Betriebsbereitschaft des Gerätes signalisieren.

Damit sich unnötige Verluste des Lösungsmittels vermeiden lassen, kann ein Auslauf des Lösungsmittel-Dosierbehälters durch eine Rücklaufleitung mit einem Vorratsbehälter für das Lössungsmittel verbunden werden. Damit liegt ein geschlossener Kreislauf vor.

Anstatt die Dosierbehälter räumlich zu trennen, können diese auch ineinandergeschachtelt werden, beispielsweise bei beengten Platzverhältnissen. Durch austauschbare Dosierbehälter lassen sich verschiedene Meßbereiche festlegen, so daß es gewünschtenfalls möglich ist, sogar unterhalb des zulässigen Toleranzbereiches liegende Meßwerte zu erhalten.

Die komplette Dosiereinheit kann als separater Bausatz mit Anschlüssen für eine Meßeinrichtung (Laborgerät) ausgeführt werden, so daß sie sich mit einer Entsorgungs- und/oder Trenn- und Aufbereitungseinrichtung in Art eines Baukastensystems zu einem kompakten Gerät zusammensetzen läßt. Die aus den beiden Behältern, den beiden Pumpen sowie Ventilen und Leitungen bestehende Dosiereinheit läßt sich beispielsweise in einen mobilen, auf Rädern verfahrbaren Schrank so einbauen, daß die von den Dosierbehältern ausgehenden Dosierleitungen unmittelbar in den darunter angeordneten Extraktor des Analyse- bzw. Laborgerätes führen bzw. sich mit Leitungen daran anschließen lassen. Unterhalb des Analysegerätes läßt sich in dem Schrank gleichzeitig auch noch die Entsorgungseinrichtung unterbringen, wobei sich die einzelnen Einheiten schubladenartig in den Schrank ein- bzw. aufsetzen lassen. Zusätzlich kann ein Drucker und ein Grenzwertschalter eingebaut bzw. angeschlossen werden, um einerseits die gemessenen Werte dokumentieren und andererseits bei Überschreiten eines Grenzwertes Alarm geben zu können.

Der Extraktor kann neben einer im Durchmesser kleinen Meßleitung eine Entleerungsleitung großen Durchmessers aufweisen, die mit einem Leitungsfortsatz bis in den Extraktor geführt wird und mit geringem Abstand oberhalb der Meßleitung endet. Hierdurch wird ein schnelles Entleeren des Extraktors gewährleistet und gleichzeitig verhindert, daß sich in der Entleerungsleitung oberhalb eines dort vorhandenen Ventils Flüssigkeit ansammeln kann, die das exakte Mischverhältnis für einen nachfolgenden Meßvorgang verfälschen könnte. Es bildet sich aufgrund der Druckverhältnisse zwischen der im Durchmesser großen Entleerungsleitung und der engeren Meßleitung oberhalb der Meßleitung in dem Leitungsfortsatz lediglich ein geringfügiger Flüssigkeitskegel bzw. Meniskus aus; die Ventile in der Meß- und Entleerungsleitung sind hierbei selbstverständlich geschlossen.

Die Erfindung wird nachfolgend anhand eines in der Zeichnung dargestellten Ausführungsbeispiels näher erläutert. In der Zeichnung zeigen:

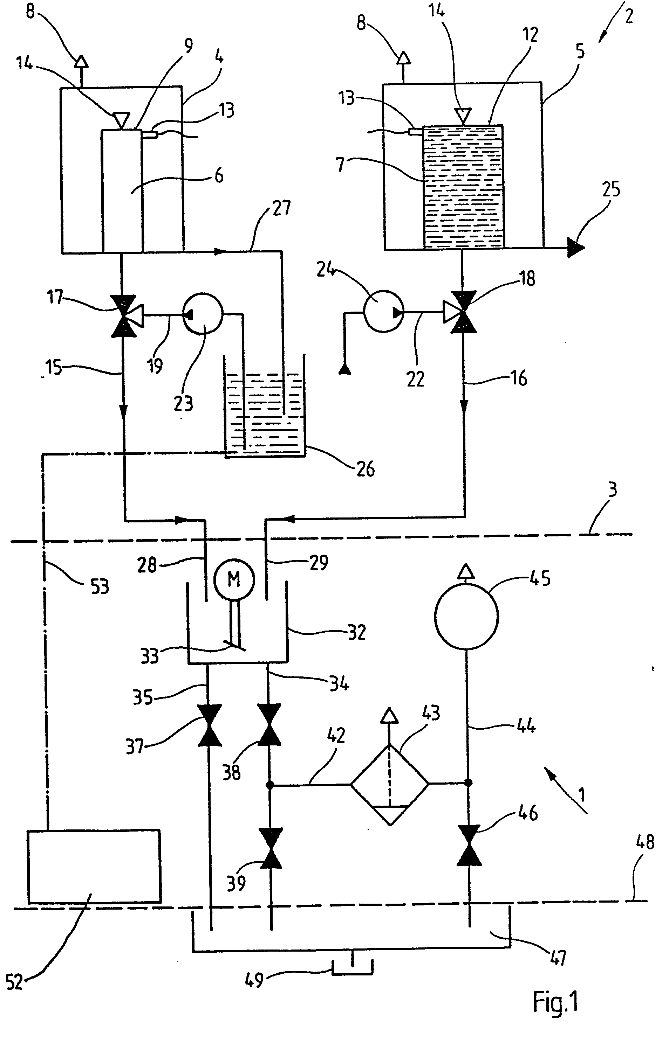

Fig. 1     eine mit einem Analyse- bzw. Laborgerät integrierte, erfindungsgemäße Dosiereinheit, schematisch dargestellt, und

Fig. 2    einen Extraktor mit einem bis knapp oberhalb einer Meßleitung reichenden Leitungsfortsatz einer
          Entleerungsleitung, schematisch dargestellt.

Die von einem üblichen Analyse- bzw. Laborgerät 1 separate
Dosiereinheit 2, was durch die gestrichelte Trennlinie 3
verdeutlicht wird, besteht im wesentlichen aus einem Dosierbehälter 4 für das Lösungsmittel und einem Dosierbehälter 5
für das Probenwasser, wobei in den Behältern 4, 5 jeweils
zylindrische Aufnahmekammern 6, 7 für die Flüssigkeiten
vorgesehen sind. Jedem Dosierbehälter 4, 5 ist eine Entlüftung 8 und im Bereich der Überlaufränder 9, 12 der oben
offenen Aufnahmekammern 6, 7 ein Näherungsschalter 13 sowie
je ein Verdrängungskegel 14 zugeordnet, der sich in die
Aufnahmekammer eintauchen läßt.

An jede Aufnahmekammer 6, 7 ist eine Dosierleitung 15, 16
mit einem darin angeordneten Mehrwegeventil 17, 18 angeschlossen. Von den Mehrwegeventilen 17, 18 gehen Zweigleitungen 19, 22 mit daran angeschlossenen Pumpen 23, 24 ab.
Während die Pumpe 24 das Probenwasser über das Drei-Wege-
Ventil 18 aus einem beliebigen, nicht dargestellten Behälter in den Dosierbehälter 5 fördert, wo es nach dem Überlaufen mittels des Ablaufs 25 entfernt wird, fördert die
Pumpe 23 das Lösungsmittel in einem geschlossenen Kreislauf. Dazu reicht die Zweigleitung 19 bis in einen Vorratsbehälter 26, wie auch eine Rücklaufleitung 27 das am Überlaufrand 9 überströmende Lösungsmittel in den Behälter 26
zurückführt.

Die Dosierleitungen 15, 16 der Dosierbehälter 4, 5 lassen
sich mittels Anschlüssen bzw. Anschlußleitungen 28, 29 mit
einem Extraktor 32 des Laborgerätes 1 verbinden. Eine motorbetriebene Taumelscheibe 33  vermischt die beiden in dem
Extraktionsgefäß zusammentreffenden Förderströme des Lösungsmittels und  des Probenwassers innig miteinander, die
sich darin völlig extrahieren lassen. Aus dem Extraktor 32

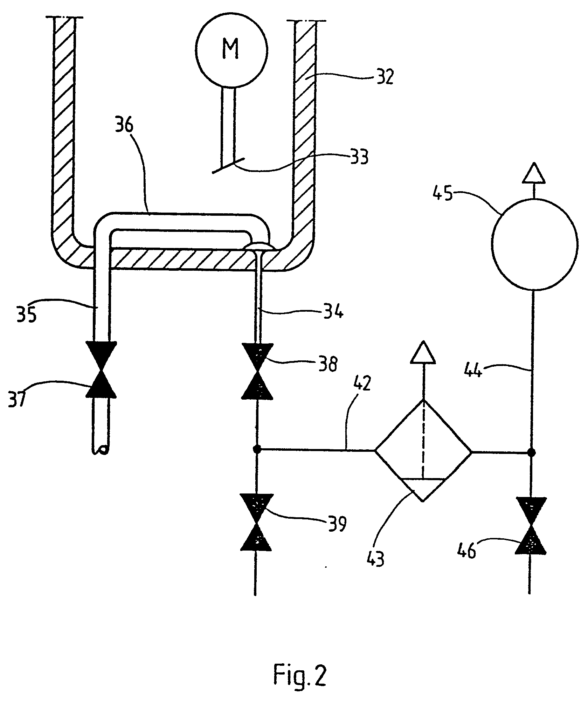

führt neben einer Meßleitung 34 geringeren Durchmessers auch eine Entleerungsleitung 35 mit einem größeren Durchmesser heraus, wobei sich im Inneren des Extraktors 32 noch ein Leitungsfortsatz 36 der Entleerungsleitung 35 erstreckt, der knapp oberhalb der Meßleitung 34 endet (Fig.2).

In der Entleerungsleitung 35 befindet sich ein steuerbares Ablaufventil 37 und in der Meßleitung 34 sind zwei steuerbare Ablaufventile 38, 39 angeordnet, von denen das Ablaufventil 38 bei abgesperrtem Ventil 39 über eine Leitung 42 die extrahierte Flüssigkeit zu einem Trennfilter 43 leitet. Im Trennfilter 43 wird vor dem Messen die Öllösung von dem Wasser abgetrennt, die dann zur Meßwertermittlung über eine Verbindungsleitung 44 zu einem Infrarot-Strahlenanalysator 45 gelangt. Nach dem Messen strömt die Öllösung über ein in der Leitung 44 angeordnetes Ablaufventil 46 in einen Auffangbehälter 47.

Der Auffangbehälter 47 und eine nicht dargestellte Entsorgungs- und/oder Trenn- und Aufbereitungseinrichtung lassen sich ebenfalls als separater Bausatz ausbilden und unterhalb der gestrichelten Trennlinie 48 mit der Dosiereinheit 2 und dem Laborgerät 1 zu einer kompakten Einheit zusammensetzen. Die dem Auffangbehälter 47 an einem Ablauf 49 zu entnehmende Flüssigkeit läßt sich aber auch extern aufbereiten und insbesondere das Lösungsmittel zurückgewinnen, wozu ein Kohlefilter 52 vorgesehen sein kann, wie schematisch dargestellt. Um einen kontinuierlichen Kreislauf und eine automatische Betriebsweise auch über längere Zeiträume zu ermöglichen, kann der Kohlefilter 52 mittels einer Versorgungsleitung 53 direkt an den Vorratsbehälter 26 für das Lösungsmittel angeschlossen werden, wie strichpunktiert dargestellt.

Beim Betrieb der Vorrichtung füllen zunächst die Pumpen 23, 24 über die Mehrwegeventile 17, 18 die Aufnahmekammern 6, 7 des Lösemittel-Dosierbehälters 4 und des Probenwasser-Dosierbehälters 5 auf, wobei Näherungsschalter 13 die Betriebsbereitschaft, d.h. den für das Messen erforderlichen Füllstand signalisieren. Beim Dosieren werden die Mehrwegeventile 17, 18 magnetbetätigt umgeschaltet, so daß einerseits die Ausgänge der Pumpen blockiert werden, d.h. kein Lösungsmittel und kein Probenwasser mehr in die Dosierbehälter gelangen kann, und andererseits die durch die Überlaufränder 9, 12 in Verbindung mit den Verdrängungskegeln 14 dosierten Flüssigkeitsmengen über die geöffneten Mehrwegeventile 17, 18 der Dosierleitungen 15, 16 in den Extraktor 32 fließen können. Der Extraktor ist dazu über die Anschlüsse 28, 29 mit den Dosierleitungen 15, 16 verbunden. Nach einem vorherigen, mehrmaligen Spülen der Leitungen erfolgt die Probennahme zur Meßwertermittlung durch Öffnen des Ablaufventils 38 bei geschlossenen Ventilen 37, 39 und 46. Der Probenstrom gelangt auf diese Weise über die Leitung 42 in den Trennfilter 43, wo sich das Wasser absetzen kann. Die Öllösung gelangt hingegen über die Verbindungsleitung 44 in das Infrarot-Strahlengerät 45, das den genauen Wert der Ölbelastung ermittelt. Die unvermeidlich kontinuierlich zunehmenden Ölrestanteile im rückgewonnenen und immer wieder neu zirkulierenden Lösungsmittel werden durch eine Nullpunktveränderung bzw. Nullpunkteinstellung des Infrarot-Strahlengerätes 45 auf das gerade verwendete Lösungsmittel berücksichtigt. Sobald der Meßwert festliegt und gegebenenfalls mittels eines Druckers aufgeschrieben worden ist, wird zunächst das Ablaufventil 46 in der Verbindungsleitung 44 zum Infrarot-Strahlenanalysators 45, danach das Ablaufventil 39 in der Meßleitung 34 und zuletzt das Ablaufventil 37 in der Entleerungsleitung 35 geöffnet.

- 8 -

Die im Extraktor 32 vorhandene Flüssigkeitsmenge kann dabei ebenfalls wie die Probenmenge des Analysators 45 in den Auffangbehälter 47 abfließen, von wo die Flüssigkeit zur erneuten Aufbereitung weitergeleitet werden kann. Danach werden die Ventile wieder geschlossen, insbesondere die Mehrwegeventile 17, 18 in den Dosierleitungen 15, 16, so daß die Pumpen 23, 24 die Dosierbehälter 4, 5 erneut mit Flüssigkeit auffüllen können.

2 br

- 9 -                                35 928 B

Pierburg GmbH & Co. KG, Leuschstraße 1,
=========================================

4040 Neuss 1
============

"Vorrichtung zum Messen des Ölgehaltes von Wasser"

Patentansprüche:

1. Vorrichtung zum Messen des Ölgehaltes von Wasser, bei dem aus einem Lösungsmittel- und einem Probenwasser-Dosierbehälter (4, 5) je eine Menge eines Lösungsmittels und eine Menge Probenwasser zu einem Extraktor (32) gefördert wird, das Öl aus dem Probenwasserstrom mit einem organischen Lösungsmittel extrahiert, das Wasser in einem Trennfilter (43) abgetrennt, das ölbeladene Lösungsmittel in einem, insbesondere als Infrarot-Meßgerät arbeitenden, Analysator (45) analysiert, das Lösungsmittel nachfolgend von dem Öl abgetrennt und im Kreislauf wiederverwendet sowie das abgetrennte Wasser über eine Reinigungsstufe abgeführt wird, dadurch gekennzeichnet, daß als Überlaufbehälter ausgebildeten Dosierbehältern (4, 5) einer Dosiereinheit (2) mit genau festgelegtem Aufnahme- bzw. Volumenverhältnis ständig umlaufende, Lösungsmittel oder Probenwasser fördernde Pumpen (23, 24) mit zwischengeschalteten, aufsteuerbaren Sperreinrichtungen (17, 18) in den Zuführleitungen (15, 19 bzw. 16, 22) zugeordnet sind.

2. Vorrichtung nach Anspruch 1, gekennzeichnet durch Mehrwegeventile (17,18) in den Zuführleitungen (15,19 bzw. 16, 22).

3. Vorrichtung nach Anspruch 1 oder 2, dadurch gekennzeichnet, daß an der Überlaufseite eines jeden Dosierbehälters (4, 5) ein Verdrängungskegel (14) in die Flüssigkeits-Aufnahmekammer (6,7) eintaucht.

4. Vorrichtung nach einem oder mehreren der Ansprüche 1 bis 3, dadurch gekennzeichnet, daß die Flüssigkeiten von Schwingankerpumpen (23, 24) gefördert werden.

5. Vorrichtung nach einem oder mehreren der Ansprüche 1 bis 4, gekennzeichnet durch Näherungsschalter (13) für die Dosierbehälter (4, 5).

6. Vorrichtung nach einem oder mehreren der Ansprüche 1 bis 5, dadurch gekennzeichnet, daß der Lösungsmittel-Dosierbehälter (4) und ein Vorratsbehälter (26) für das Lösungsmittel durch eine Rücklaufleitung (27) miteinander verbunden sind.

7. Vorrichtung nach einem oder mehreren der Ansprüche 1 bis 6, gekennzeichnet durch ineinandergeschachtelte Dosierbehälter (4, 5).

8. Vorrichtung nach einem oder mehreren der Ansprüche 1 bis 7, gekennzeichnet durch austauschbare Dosierbehälter (4, 5).

9. Vorrichtung nach einem oder mehreren der Ansprüche 1 bis 8, dadurch gekennzeichnet, daß die komplette Dosiereinheit (2) als separater Bausatz mit Anschlüssen (28, 29) für eine Meßeinrichtung (Laborgerät) (1) ausgeführt ist.

10. Vorrichtung nach einem oder mehreren der Ansprüche 1 bis 9, dadurch gekennzeichnet, daß der Extraktor (32) neben einer im Durchmesser kleinen Meßleitung (34) eine Entleerungsleitung (35) großen Durchmessers aufweist, die mit einem Leitungsfortsatz (36) bis in den Extraktor (32) geführt wird und mit geringem Abstand oberhalb der Meßleitung (34) endet.

0160815

Fig.1

Fig.2